# EUROPEAN PATENT SPECIFICATION

(11) **EP 4 269 386 B1**
(45) Date of publication and mention of the grant of the patent: **26.11.2025**
(21) Application number: 23168646.0
(22) Date of filing: 19.04.2023
(51) Int. Cl.: C07C 233/47, C07C 231/02, C07C 231/24

(54) **A METHOD FOR PRODUCING A HIGHLY PURIFIED SALT OF N-ACYL NEUTRAL AMINO ACID AND AN AQUEOUS COMPOSITION OF HIGHLY PURIFIED SALT OF N-ACYL AMINO ACID OBTAINED WITH THIS METHOD**
VERFAHREN ZUR HERSTELLUNG EINES HOCHREINEN SALZES AUS N-ACYL NEUTRALER AMINOSÄURE UND MIT DIESEM VERFAHREN ERHALTENE WÄSSRIGE ZUSAMMENSETZUNG
PROCÉDÉ DE PRODUCTION D'UN SEL D'ACIDE AMINÉ NEUTRE N-ACYLE HAUTEMENT PURIFIÉ ET COMPOSITION AQUEUSE D'UN SEL D'ACIDE AMINÉ N-ACYLE HAUTEMENT PURIFIÉ OBTENUE PAR CE PROCÉDÉ

(30) Priority: 26.04.2022 PL 44102222
(43) Date of publication of application: 01.11.2023
(73) Proprietor: PCC EXOL S.A., 56-120 Brzeg Dolny (PL)
(72) Inventor: Frackowiak, Renata, 51-211 Wroclaw (PL); Kucharczyk, Sylwia, 54-060 Wroclaw (PL)
(74) Representative: JWP Patent & Trademark Attorneys

(56) References cited:
- EP-B1- 0 857 717
- US-A- 5 710 295
- US-A- 5 985 798
- US-B1- 6 569 829
- US-B2- 6 703 517

## Description

The present invention relates to a method for producing a highly purified surfactant of N-acyl neutral amino acid by using an acidic form as a transitional form and an aqueous composition of highly purified salt of N-acyl neutral amino acid obtained with this method. Amino acid surfactants belonging to a group of anionic surface active agents are produced on the basis of biomimetics, i.e. raw materials imitating naturally occurring chemical compounds and fatty acids, i.e. raw materials of plant origin. Surfactants of this type are most often found in the form of an aqueous solution and are widely used in various industries (in cosmetics, detergents, household or industrial cleaning agents). Owing to the removal of NaCl, this product can be successfully used as a corrosion inhibitor or an ingredient of cosmetic compositions, in which NaCl is an undesirable ingredient.

Sarcosinates are the most abundant group of amino acid surfactants. Due to their very good foam producing properties, antibacterial and wetting properties, no hydrolysis in the alkaline environment, they are used in the production of foodstuffs, in the cosmetic industry (shampoos, toothpastes), carpet cleaning agents, car care products or cleaning and disinfecting agents.

In the agrochemical industry, sarcosinates are predominantly used as glyphosate adjuvants, as disclosed in patent application US 5985798A.

In a desalted form, they are mainly used as corrosion inhibitors in various process fluids, such as lubricating oils and solid lubricants, hydraulic fluids, fluids for drilling and cutting, distilled fuels, etc.

Patent US 5710295 describes a process of preparing alkali metal N-acyl amino acids, particularly sodium N-acyl sarcosinate. The method shown therein involves reacting an amino acid salt directly with a fatty acid at elevated temperatures, with generated water being constantly collected.

The most traditional method for producing amino acid surfactants is based on the Schotten-Baumann reaction. Patent application US 2004/0063980 A1 describes a method for producing N-acyl amino acids, comprising the following steps: placing a mixture consisting of at least one amino acid or amino acid salt, a hydroxide of alkaline earth metal salt, and the addition of a fatty acid halide in a reactor. The fatty acid and in particular the fatty acid halide reacts in water and/or water-soluble organic solvent together with the amino acid. According to the presented method, N-acyl amino acids with a content of NaCl as an inorganic impurity in the range of 3-10% are obtained.

Patent EP 0827950 B1 describes the synthesis of an N-long chain acyl acidic amino acid or a salt thereof by condensing the acidic amino acid or a salt thereof with a fatty acid chloride in an aqueous solution without an additional organic solvent.

Patent EP 0857717 B1 discloses a process for producing an N-long chain acyl amino acid by reacting an acidic amino acid with a long chain fatty acid halide in water in the presence of an alkali and a polyhydric alcohol. Patent document US 6569829 B1 relates to a method for producing an amino acid surfactant by reacting an acidic amino acid with long chain fatty acid halides in a mixture of water and tertiary alcohol as solvents. The method described therein comprises a step of precipitating fatty acid N-acylated acidic amino acid with a mineral acid, a step of washing the product with a mixture of water and tert-butanol as solvents, as well as a step of neutralising and distilling the solvents. The product is purified to remove inorganic impurities by separating aqueous and organic phases at a temperature of 35°C to 80°C.

Patent document US 6703517 B2 describes a method for preparing a surfactant belonging to a group of neutral amino acids such as glycine, gamma-aminobutyric acid, or alanine, with a saturated or unsaturated fatty acid halide having 8 to 22 carbon atoms, wherein the reaction is carried out in a mixture of water and one or more kinds of hydrophilic organic solvents selected from a group consisting of acetone, acetonitrile, a secondary alcohol having 3 or 4 carbon atoms, and a tertiary alcohol having 4 carbon atoms in the presence of a base. The phase separation process is carried out at a temperature above 35°C, and the most preferred solvents ensuring the separation of the organic layer from the aqueous layer are isopropanol, sec-butanol and *tert*-butanol*.* The documents known in the state of the art focus mostly on the process of obtaining a purified form of N-acyl amino acids and specify that the process is required to be carried out at a temperature above 35°C under highly acidic conditions (pH 1-4). Under such conditions, the main product may decompose through its acid hydrolysis and, consequently, by-products may form, leading to a decrease in the purity of the final compound and thus to a decrease in the efficiency of the entire process. This is particularly important when using N-acyl amino acids as corrosion inhibitors or as ingredients of cosmetic compositions, in which any impurities, and in particular NaCl, are an undesirable ingredient.

An object of the invention is to improve the process of obtaining salts of N-acyl neutral amino acids and to eliminate the aforementioned drawbacks and defects of the methods used so far by developing a method for preparing highly pure salts, in particular free from inorganic impurities such as alkali metal halides, such as e.g., NaCl.

An object of the invention is a method for producing a highly purified salt of an N-acyl neutral amino acid, in which, in the first step, a neutral amino acid is reacted with a halide of an organic acid comprising 8 to 22 carbon atoms, in water as a solvent, at pH 10-11 and at a temperature of 15-25°C, the resulting mixture is then heated to a temperature of ≤ 80°C, then cooled down to room temperature and an organic solvent and an inorganic acid solution are added to pH ≤ 3, while maintaining the temperature of the mixture below 30°C. In the next step, the resulting layers are separated into an aqueous phase containing impurities and an organic phase containing an acidic form of an N-acyl neutral amino acid and the organic phase is evaporated. The last step consists in alkalising the acidic form of N-acyl neutral amino acid by dissolving it in alkaline water to obtain the pH of the solution in the range of 8-10.

Preferably, the organic acid halide is a fatty acid halide, more preferably a saturated fatty acid halide.

Preferably, the organic acid halide is an organic acid chloride.

Preferably, the salt of the N-acyl neutral amino acid is a sodium salt of N-acyl neutral amino acid.

Preferably, the sodium salt of the N-acyl neutral amino acid is a sodium salt of N-lauroyl sarcosine acid.

Preferably, the organic solvent from step b) is a mixture of two or more organic solvents. More preferably, the organic solvent is a hydrophilic organic solvent or a mixture of hydrophilic organic solvents.

More preferably, the hydrophilic organic solvent is selected from the group of monohydroxy alcohols.

Most preferably, the hydrophilic organic solvent is at least one hydrophilic organic solvent selected from a group comprising methanol, ethanol, isopropanol, sec-butanol and *tert-*butanol.

Preferably, the ratio of the aqueous solution of the N-acyl neutral amino acid salt to the organic solvent is from 1:1 to 1:5, preferably from 1:1 to 1:3.

Preferably, the inorganic acid from step b) is sulphuric(VI) acid.

Preferably, the concentration of inorganic acid is from 10 to 30% w/w.

Preferably, the separation temperature in step c) is between 18 and 30°C.

Preferably, in steps a) and d) an aqueous solution of alkaline earth hydroxide is used as an alkalinizing agent.

Preferably, the aqueous solution of alkaline earth hydroxide is an aqueous solution of sodium hydroxide, preferably at a concentration of less than or equal to 50%.

Preferably, the neutral amino acid is glycine and sarcosine.

A further object of the invention is to provide a method for producing an aqueous composition of a highly purified salt of N-acyl neutral amino acid, preferably glycine and sarcosine, characterised in that the content of inorganic impurities is less than 1%.

Preferably, the content of inorganic impurities is less than 0.4%.

Preferably, the inorganic impurity is NaCl.

Preferably, the pH of 10% aqueous composition is less than or equal to 10, and the colour of the 10% aqueous composition on the Hazen scale is less than or equal to 20. An advantage of the present invention is that N-acyl neutral amino acids are obtained in which contamination with alkali metal halides is less than 1% by weight of the mixture, which is particularly important when using these compounds as corrosion inhibitors or as ingredients of cosmetic compositions.

A further advantage of the present invention is that the purification process of N-acyl neutral amino acids is carried out under mild conditions, thus avoiding the risk of acid hydrolysis of the product, which leads to an increase in the total yield of the products obtained, as they are obtained with the yields of 90-95%. The method used is therefore more effective compared to other known methods and helps obtain products with a lower content of impurities.

With the method of the invention, a highly purified aqueous N-acyl amino acid composition free from impurities in the form of organic and inorganic solvents in the amount of less than 1% is also produced.

The following examples are illustrative of the present invention without limiting its scope. For the qualitative analysis of the final products, the following were used in the examples below: dry matter as determined with a moisture balance, pH as determined with a SevenEasy pH meter from Mettler-Toledo, NaCl content as determined by potentiometric titration with silver nitrate, the amount of water as determined by a Karl-Fischer volumetric analysis and the colour as determined on the Hazen scale by spectrophotometry with a spectrophotometer for measuring absorbance from Hunter.

### Example 1

### a) Acylation stage

158g 40% aqueous solution of sarcosine sodium salt was placed in a jacketed reactor connected to a cryostat equipped with a stirring rod along with an end, a dropper with lauroyl chloride and a dropper with 50% NaOH, 369g demineralized water and 45.5g 50% NaOH were added. The stirrer was started and the whole was homogenized. The temperature of the mixture was set at 15°C and its pH at 12. Slow dosing of 127.5g lauroyl chloride was started and continued for 4h, while maintaining the temperature in the range of 15-25°C and the pH in the range of 10.0-11.0. If necessary, the pH was adjusted by dosing soda lye (50% solution). After the lauric acid chloride had been dosed, the whole was stirred under the same conditions for additional 1.5 hours. It was then heated to 60°C and the batch was heated for additional 1.5 hours. After this time, the reactor was cooled down to 30°C and discharged. The final outcome was a product being a sodium salt of N-lauroyl sarcosine acid (Rokatend LS) with an NaCl salt content of 4.9%, dry matter of 29.4%, water content of 69.5%, pH of 10.1 for a 10% solution and colour 20 on the Hazen scale.

### b) Purification step

A 600g sample of N-lauroyl sarcosine acid sodium salt (Rokatend LS) was placed in a glass reactor equipped with a magnetic dipole and placed on a magnetic stirrer, 200g isopropanol was added and, in the next step, 113g 25% H₂SO₄ was added to obtain the mixture pH below 3. The pH was 2.4. The mixture became cloudy and opalescent. The whole was stirred at a temperature of 20°C for 1h. The mixture was then transferred to a separatory funnel and after 20 minutes the two resulting phases were completely separated. The bottom layer was poured into a bottle to obtain 587.14g of an aqueous layer. The top layer in the amount of 325.8g was transferred to a flask to evaporate isopropanol. The isopropanol was evaporated on a vacuum evaporator at a temperature of 80°C at a pressure of 50-100mbar for 30 minutes to obtain 135.1g of a substance in the form of oil, with a dry matter of about 95%, which is mainly N-acyl sarcosine acid. The oily substance obtained was then dissolved in a mixture of 312.4g water and 40g 50% NaOH solution to obtain as a final outcome a product with an NaCl salt content of 0.13%, dry matter of 30%, water content of 69.2%, pH of 8.3 for a 10% solution and colour 20 on the Hazen scale.

### Example 2

A 600g sample of Rokatend LS obtained according to Example 1 a) was placed in a glass reactor equipped with a magnetic dipole and placed on a magnetic stirrer, and 50g pure isopropanol and 150g distillate with the isopropanol from the previous purification process were added. Then in the next step 113g 25% H₂SO₄ was added to obtain the mixture pH below the 3. The pH was 2.8. The mixture at that point became cloudy and opalescent. The whole was stirred at a temperature of 18°C for 1h. The mixture was then transferred to a separatory funnel and after 20 minutes the two resulting phases were completely separated. The bottom layer was poured into a bottle to obtain 669.27g of an aqueous layer. The top layer in the amount of 238.05g was transferred to a flask to evaporate isopropanol. The isopropanol was evaporated on a vacuum evaporator at a temperature of 80°C at a pressure of 50-100mbar for 30 minutes to give 132.63g of a substance with an oil-like consistence, with a dry matter of about 95%, which is mainly N-acyl sarcosine acid. The oily substance obtained was dissolved in a mixture of 312g water and 40g 50% NaOH solution to obtain as a final outcome a product with an NaCl salt content of 0.18%, dry matter of 29.7%, water content of 70.5%, pH of 8.7 for a 10% solution and colour 10 on the Hazen scale.

### Example 3

A 600g sample of Rokatend LS obtained according to Example 1 a) was placed in a glass reactor equipped with a magnetic dipole and placed on a magnetic stirrer, 200g isopropanol was added and, in the next step, 81.77g 25% H₂SO₄ was added. The pH was 4.0. The mixture at that point became cloudy and opalescent. The whole was stirred at a temperature of 25°C for 1h. The mixture was then transferred to a separatory funnel and after 20 minutes the two resulting phases were completely separated. The bottom layer was poured into a bottle to obtain 571.88g of an aqueous layer. The top layer in the amount of 294.41g was transferred to a flask to evaporate isopropanol. The isopropanol was evaporated on a vacuum evaporator at a temperature of 80°C at a pressure of 50-100mbar for 30 minutes to give 133.28g of a substance with an oil-like consistence, with a dry matter of about 95%, which is mainly N-acyl sarcosine acid. The oily substance obtained was dissolved in a mixture of 312.4g water and 40g 50% NaOH solution to obtain as a final outcome a product with an NaCl salt content of 1%, dry matter of 29.9%, water content of 70.6%, pH of 10.1 for a 10% solution and colour 20 on the Hazen scale. As can be seen, as the pH during the acidification of the sample was too high, a valuable product with a low NaCl content was not obtained.

### Example 4

A 600g sample of Rokatend LS obtained according to Example 1 a) was placed in a glass reactor equipped with a magnetic dipole and placed on a magnetic stirrer, 200g ethanol was added and, in the next step, 140g 25% H₂SO₄ was added. The pH was 2.2. The mixture at that point became cloudy and opalescent. The whole was stirred at a temperature of 25°C for 1h. The mixture was then transferred to a separatory funnel and after 60 minutes the two resulting phases were completely separated. The bottom layer was poured into a bottle to obtain 636g of an aqueous layer. The top layer in the amount of 291g was transferred to a flask to evaporate ethanol. The ethanol was evaporated on a vacuum evaporator at a temperature of 70°C at a pressure of 50-100mbar for 30 minutes to give 138g of a substance with an oil-like consistency, with a dry matter of about 95%, which is mainly N-acyl sarcosine acid. The oily substance obtained was dissolved in a mixture of 312.4g water and 40g 50% NaOH solution to obtain as a final outcome a product with an NaCl salt content of 0.26%, dry matter of 30.8%, water content of 69.2%, pH of 9.5 for a 10% solution and colour 10 on the Hazen scale. During the process, the phase separation stage was less efficient than with the use of isopropanol, as ethanol mixes with water to a greater extent, which makes the phase separation significantly longer.

### Example 5

A 550g sample of Rokatend LS obtained according to Example 1 a) was placed in a glass reactor equipped with a magnetic dipole and placed on a magnetic stirrer, 180g methanol and 120g 25% H₂SO₄ were added. The pH was 3. The mixture at that point became cloudy and opalescent. The whole was stirred at a temperature of 25°C for 1h. The mixture was then transferred to a separatory funnel and after 60 minutes the two resulting phases were completely separated. The bottom layer was poured into a bottle to obtain 650g of an aqueous layer. The top layer in the amount of 200g was transferred to a flask to evaporate methanol. The methanol was evaporated on a vacuum evaporator at a temperature of 70°C at a pressure of 50-100mbar for 30 minutes to give 90g of a substance with an oil-like consistency, with a dry matter of about 90%, which is mainly N-acyl sarcosine acid. The oily substance obtained was dissolved in a mixture of 262g water and 30g 50% NaOH solution to obtain as a final outcome a product with an NaCl salt content of 1.5%, dry matter of 29%, water content of 70%, pH of 9.5 for a 10% solution and colour 10 on the Hazen scale. During the process, the phase separation step was less efficient than with the use of isopropanol, as methanol mixes with water to a greater extent, thus the phase separation is significantly longer, and more active substance remains in the aqueous solution.

### Example 6

A 600g sample of Rokatend LS obtained according to Example 1 a) was placed in a glass reactor equipped with a magnetic dipole and placed on a magnetic stirrer, 200g isopropanol was added and, in the next step, 113g 25% H₂SO₄ was added. The pH was 2.7. The mixture at that point became cloudy and opalescent. The whole was stirred at a temperature of 40°C for 1h. The mixture was then transferred to a separatory funnel and after 20 minutes the two resulting phases were completely separated. The bottom layer was poured into a bottle to obtain 572g of an aqueous layer. The top layer in the amount of 379g was transferred to a flask to evaporate isopropanol. The isopropanol was evaporated on a vacuum evaporator at a temperature of 80°C at a pressure of 50-100mbar for 30 minutes to give 102g of a substance with an oil-like consistence, with a dry matter of about 95%, which is mainly N-acyl sarcosine acid. The resulting oily substance was dissolved in a mixture of 312.4g water and 40g 50% NaOH solution to obtain as a final outcome a product with an NaCl salt content of 0.4%, dry matter of 30.5%, water content of 69.3% and pH for a 10% solution of 10.1. As can be seen, the higher temperature led to a significantly deteriorated phase separation, which can be seen in the amount of the aqueous and organic layers obtained as compared to the process carried out at a temperature below 35°C. A much worse purification effect was also obtained, as the amount of NaCl in the final product was 0.4%.

The embodiments illustrate the relationship between the mixture pH and the temperature at which the purification step is carried out with the amount of other inorganic impurities in the aqueous composition of *N*-acyl amino acid salt. The performance of the purification step at a pH higher than 3 or at a temperature above 30°C results in an aqueous composition of *N*-acyl amino acid salt with an unfavourable NaCl content of ≥0.4%.

The method according to the invention is particularly advantageous from the point of view of green chemistry and constantly increasing environmental pollution. According to the invention, no toxic catalysts or halogenated solvents are used, which plays a significant part in the amount of harmful chemical waste generated. In addition, the purification step is very advantageous in terms of cost-effectiveness as being carried out at room temperature it requires no supply of additional energy to the system, which would entail an increase in the costs of the entire process.

## Claims

1. A method for producing a highly purified salt of N-acyl neutral amino acid, comprising the following steps:
a. a neutral amino acid is reacted with a halide of an organic acid comprising from 8 to 22 carbon atoms, in water as a solvent, at a pH of 10-11 and a temperature of 15-25°C;
b. the mixture is heated to a temperature of ≤ 80°C, then the mixture is cooled down to room temperature and an organic solvent and an inorganic acid solution are added to pH ≤ 3, while maintaining the temperature of the mixture below 30°C;
c. the resulting layers are separated into an aqueous phase containing impurities and an organic phase containing an acidic form of N-acyl neutral amino acid and the organic phase is evaporated;
d. the acidic form of the N-acyl neutral amino acid is alkalised by dissolving it in alkaline water to obtain the pH of the solution in the range of 8-10.

2. The method of claim 1, **characterised in that** the organic acid halide is a fatty acid halide, **preferably** a saturated fatty acid halide.

3. The method of claim 1 or 2, **characterised in that** the organic acid halide is an organic acid chloride.

4. The method of claim 1, **characterised in that** the salt of N-acyl neutral amino acid is a sodium salt of N-acyl neutral amino acid.

5. The method of claim 4, **characterised in that** the sodium salt of N-acyl neutral amino acid is a sodium salt of N-lauroyl sarcosine acid.

6. The method of claim 1, **characterised in that** the organic solvent from step b) is a mixture of two or more organic solvents.

7. The method of claim 6, **characterised in that** the organic solvent is a hydrophilic solvent or a mixture of hydrophilic organic solvents.

8. The method of claim 7, **characterised in that** the hydrophilic organic solvent is selected from a group of monohydroxy alcohols.

9. The method of claim 8, **characterised in that** the hydrophilic organic solvent is at least one hydrophilic organic solvent selected from a group comprising methanol, ethanol, isopropanol, sec-butanol and *tert*-butanol.

10. The method of claim 1, **characterised in that** the ratio of an aqueous solution of N-acyl neutral amino acid salt to the organic solvent is from 1:1 to 1:5, **preferably** from 1:1 to 1:3.

11. The method of claim 1, **characterised in that** the inorganic acid from step b) is sulphuric(VI) acid.

12. The method of claim 11, **characterised in that** the concentration of inorganic acid is from 10 to 30% w/w.

13. The method of claim 1, **characterised in that** the separation temperature in step b) is between 18 and 30°C.

14. The method of claim 1, **characterised in that** in step a) and d) an aqueous solution of alkaline earth hydroxide is used as an alkalinizing agent.

15. The method of claim 14, **characterised in that** the aqueous solution of alkaline earth hydroxide is an aqueous solution of sodium hydroxide, **preferably** at a concentration of less than or equal to 50%.

16. The method of claim 1, **characterised in that** the neutral amino acid is glycine, sarcosine, tyrosine, and taurine.

## Patentansprüche

1. Verfahren zur Herstellung eines hochgereinigten Salzes einer neutralen N-Acylaminosäure, das die folgenden Schritte umfasst:
a. eine neutrale Aminosäure wird mit einem Halogenid einer organischen Säure, die 8 bis 22 Kohlenstoffatome umfasst, in Wasser als Lösungsmittel bei einem pH-Wert von 10-11 und einer Temperatur von 15-25°C umgesetzt;
b. die Mischung wird auf eine Temperatur von ≤ 80°C erhitzt, dann wird die Mischung auf Raumtemperatur abgekühlt und ein organisches Lösungsmittel und eine anorganische Säurelösung werden bis zu einem pH-Wert von ≤ 3 hinzugefügt, während die Temperatur der Mischung unter 30°C gehalten wird;
c. die resultierenden Schichten werden in eine wässrige Phase, die Verunreinigungen enthält, und eine organische Phase, die eine saure Form einer neutralen N-Acylaminosäure enthält, getrennt und die organische Phase wird eingedampft;
d. die saure Form der neutralen N-Acylaminosäure wird durch Auflösen in alkalischem Wasser alkalisiert, um den pH-Wert der Lösung im Bereich von 8-10 zu erreichen.

2. Verfahren nach Anspruch 1, **dadurch gekennzeichnet, dass** das organische Säurehalogenid ein Fettsäurehalogenid, **vorzugsweise** ein gesättigtes Fettsäurehalogenid ist.

3. Verfahren nach Anspruch 1 oder 2, **dadurch gekennzeichnet, dass** das organische Säurehalogenid ein organisches Säurechlorid ist.

4. Verfahren nach Anspruch 1, **dadurch gekennzeichnet, dass** das Salz der neutralen *N*-Acylaminosäure ein Natriumsalz einer neutralen *N*-Acylaminosäure ist.

5. Verfahren nach Anspruch 4, **dadurch gekennzeichnet, dass** das Natriumsalz der neutralen *N*-Acylaminosäure ein Natriumsalz der *N*-Lauroylsarcosinsäure ist.

6. Verfahren nach Anspruch 1, **dadurch gekennzeichnet, dass** das organische Lösungsmittel aus Schritt b) eine Mischung aus zwei oder mehreren organischen Lösungsmitteln ist.

7. Verfahren nach Anspruch 6, **dadurch gekennzeichnet, dass** das organische Lösungsmittel ein hydrophiles Lösungsmittel oder eine Mischung hydrophiler organischer Lösungsmittel ist.

8. Verfahren nach Anspruch 7, **dadurch gekennzeichnet, dass** das hydrophile organische Lösungsmittel aus einer Gruppe von Monohydroxyalkoholen ausgewählt wird.

9. Verfahren nach Anspruch 8, **dadurch gekennzeichnet, dass** das hydrophile organische Lösungsmittel mindestens ein hydrophiles organisches Lösungsmittel ist, ausgewählt aus einer Gruppe umfassend Methanol, Ethanol, Isopropanol, sec-Butanol und tert-Butanol.

10. Verfahren nach Anspruch 1, **dadurch gekennzeichnet, dass** das Verhältnis einer wässrigen Lösung des Salzes einer neutralen *N*-Acylaminosäure zum organischen Lösungsmittel von 1:1 bis 1:5, **vorzugsweise** von 1:1 bis 1:3 beträgt.

11. Verfahren nach Anspruch 1, **dadurch gekennzeichnet, dass** die anorganische Säure aus Schritt b) Schwefel(VI)-säure ist.

12. Verfahren nach Anspruch 11, **dadurch gekennzeichnet, dass** die Konzentration der anorganischen Säure 10 bis 30% Gew./Gew. beträgt.

13. Verfahren nach Anspruch 1, **dadurch gekennzeichnet, dass** die Trenntemperatur in Schritt b) zwischen 18 und 30°C ist.

14. Verfahren nach Anspruch 1, **dadurch gekennzeichnet, dass** in Schritt a) und d) eine wässrige Lösung von Erdalkalihydroxid als Alkalisierungsmittel verwendet wird.

15. Verfahren nach Anspruch 14, **dadurch gekennzeichnet, dass** die wässrige Lösung von Erdalkalihydroxid eine wässrige Lösung von Natriumhydroxid ist, **vorzugsweise** in einer Konzentration von weniger als oder gleich 50%.

16. Verfahren nach Anspruch 1, **dadurch gekennzeichnet, dass** die neutrale Aminosäure Glycin, Sarcosin, Tyrosin und Taurin ist.

## Revendications

1. Procédé pour la production d'un sel hautement purifié d'acide aminé neutre N-acylé, comprenant les étapes suivantes:
a. un acide aminé neutre est mis à réagir avec un halogénure d'un acide organique comprenant de 8 à 22 atomes de carbone, dans l'eau comme solvant, à un pH de 10-11 et une température de 15-25°C;
b. le mélange est chauffé à une température ≤ 80°C, puis le mélange est refroidi à température ambiante et un solvant organique et une solution d'acide inorganique sont ajoutés jusqu'à pH ≤ 3, tout en maintenant la température du mélange en dessous de 30°C;
c. les couches résultantes sont séparées en une phase aqueuse contenant des impuretés et une phase organique contenant une forme acide d'acide aminé neutre *N*-acylé et la phase organique est évaporée;
d. la forme acide de l'acide aminé neutre *N*-acylé est alcalinisée en la dissolvant dans de l'eau alcaline pour obtenir le pH de la solution dans la plage de 8 à 10.

2. Procédé selon la revendication 1, **caractérisé en ce que** l'halogénure d'acide organique est un halogénure d'acide gras, **de préférence** un halogénure d'acide gras saturé.

3. Procédé selon la revendication 1 ou 2, **caractérisé en ce que** l'halogénure d'acide organique est un chlorure d'acide organique.

4. Procédé selon la revendication 1, **caractérisé en ce que** le sel d' acide aminé neutre *N*-acylé est un sel de sodium d'acide aminé neutre *N*-acylé.

5. Procédé selon la revendication 4, **caractérisé en ce que que** le sel de sodium de l'acide aminé neutre *N*-acylé est un sel de sodium de l'acide du *N*-lauroyl sarcosine.

6. Procédé selon la revendication 1, **caractérisé en ce que** le solvant organique de l'étape b) est un mélange de deux ou plusieurs solvants organiques.

7. Procédé selon la revendication 6, **caractérisé en ce que** le solvant organique est un solvant hydrophile ou un mélange de solvants organiques hydrophiles.

8. Procédé selon la revendication 7, **caractérisé en ce que** le solvant organique hydrophile est choisi dans un groupe d'alcools monohydroxylés.

9. Procédé selon la revendication 8, **caractérisé en ce que** le solvant organique hydrophile est au moins un solvant organique hydrophile choisi dans un groupe comprenant le méthanol, l'éthanol, l'isopropanol, le *sec*-butanol et le *tert*-butanol.

10. Procédé selon la revendication 1, **caractérisé en ce que** le rapport d'une solution aqueuse de sel d'acide aminé neutre *N*-acylé au solvant organique est de 1:1 à 1:5, **de préférence** de 1:1 à 1:3.

11. Procédé selon la revendication 1, **caractérisé en ce que** l'acide inorganique de l'étape b) est l'acide sulfurique (VI).

12. Procédé selon la revendication 11, **caractérisé en ce que** la concentration en acide inorganique est de 10 à 30 % p/p.

13. Procédé selon la revendication 1, **caractérisé en ce que** la température de séparation à l'étape b) est comprise entre 18 et 30°C.

14. Procédé selon la revendication 1, **caractérisé en ce que** dans les étapes a) et d) on utilise comme agent alcalinisant une solution aqueuse d'hydroxyde alcalino-terreux.

15. Procédé selon la revendication 14, **caractérisé en ce que** la solution aqueuse d'hydroxyde alcalino-terreux est une solution aqueuse d'hydroxyde de sodium, **de préférence** à une concentration inférieure ou égale à 50 %.

16. Procédé selon la revendication 1, **caractérisé en ce que** l'acide aminé neutre est la glycine, la sarcosine, la tyrosine et la taurine.
